# EUROPEAN PATENT SPECIFICATION

(11) **EP 3 906 849 B1**
(45) Date of publication and mention of the grant of the patent: **06.03.2024**
(21) Application number: 21172521.3
(22) Date of filing: 06.05.2021
(51) Int. Cl.: A61B 5/024, A61B 5/0245, A61B 5/0507, A61B 5/00, A61B 5/268, A61B 5/308, A61B 5/327, A61B 5/11, A61B 5/0205, A61B 5/117, A47K 13/00

(54) **METHOD AND SYSTEM FOR CONTINUOUS BIOMETRIC RECOGNITION AND/OR BIOMEDICAL ASSESSMENT IN SANITATION FACILITIES**
VERFAHREN UND SYSTEM ZUR KONTINUIERLICHEN BIOMETRISCHEN ERKENNUNG UND/ODER BIOMEDIZINISCHEN BEURTEILUNG IN SANITÄREINRICHTUNGEN
PROCÉDÉ ET SYSTÈME DE RECONNAISSANCE BIOMÉTRIQUE CONTINUE ET/OU D'ÉVALUATION BIOMÉDICALE DANS DES INSTALLATIONS SANITAIRES

(30) Priority: 06.05.2020 IT 202000010093
(43) Date of publication of application: 10.11.2021
(73) Proprietor: OLI - SISTEMAS SANITARIOS, S.A., 3800-314 Aveiro (PT)
(72) Inventor: MOURA DE OLIVEIRA, Antonio Manuel, 3800-314 AVEIRO (PT); RODRIGUES DE ALMEIDA, Hugo Márcio, 3800-314 AVEIRO (PT); PLÁCIDO DA SILVA, Hugo Humberto, 3800-314 AVEIRO (PT)
(74) Representative: Studio Torta S.p.A.

(56) References cited:
- CN-A- 106 930 380
- US-A1- 2014 361 871
- US-A1- 2016 374 619
- US-A1- 2017 188 864
- BRUSER CHRISTOPH ET AL: "Ambient and Unobtrusive Cardiorespiratory Monitoring Techniques", IEEE REVIEWS IN BIOMEDICAL ENGINEERING, vol. 8, 14 August 2015 (2015-08-14), pages 30-43, XP011666728, ISSN: 1937-3333, DOI: 10.1109/RBME.2015.2414661 [retrieved on 2015-08-17]

## Description

### CROSS-REFERENCE TO RELATED APPLICATIONS

This Patent Application claims priority from Italian Patent Application No. 102020000010093 filed on May 6, 2020.

### TECHNICAL FIELD

The present invention relates to a method and a system for continuous biometric recognition and/or biomedical assessment in sanitation facilities; in particular, the invention relates to a method and a system for detecting biomedical parameters from a subject seated on a toilet seat.

### BACKGROUND ART

Biomedical sensing has greatly evolved from traditional monolithical clinical setups, and many features can now be found in the form of wrist-worn wearable computers (aka wearables). Despite the rapid growth, wearables suffer from high abandon rates and are often described as a fad (or novelty item). Furthermore, users need to remember wearing the devices, charging them, and styling can be an issue too. As an alternative or as a complement, new approaches for biomedical sensing where no voluntary action from the user is needed are of utmost importance.

Therefore, so-called "off-the-person" (or "invisible") approach has been also proposed, based on the use of sensors which are integrated in everyday use objects. One important aspect in this approach is the mapping between the collected data and the monitored user, i.e. identification or authentication. The former are generally framed in the field of biometric recognition. Typical biometric traits are only suitable for momentary validation, bound to a short period of time, and require either recall (e.g. password), a token (e.g. identity card), or a physical (e.g. fingerprint) or behavioral (e.g. writing patterns) token from the user.

In a continuous biometric recognition and biomedical assessment paradigm, there are multiple constraints currently unmet within the state of the art. Especially, few practical solutions are known within the state-of-the-art that provide an effective end-to-end solution for this purpose. Nevertheless, several examples of systems that provide biomedical assessment in sanitation facilities, in particular associated with toilet seats, can already be found.

For example, US2016374619A1 discloses a system and a method for medical analysis of a subject seated on a toilet seat.

Other examples of similar systems and methods are provided by WO2005070288A1 and WO2018180398A1.

All the above mentioned prior art documents fail however to provide a fully effective and reliable system for detecting biomedical parameters from a subject, in particular without requiring an active intervention by the subject and at the same time ensuring the correct identification of the subject.

In fact, the prior art systems generally suffer from some drawbacks: for example, some devices are not able to provide a reliable identification of the users; and/or require several sensors and detection terminals that interface with the body of the user; and/or provide some parameters with a low level of accuracy and reliability; etc.

### DISCLOSURE OF INVENTION

It is an object of the present invention to provide a system and method for detecting biomedical parameters from a subject seated on a toilet seat without the described drawbacks of the known art.

In particular, it is an object of the invention to provide a system and method which provide reliable identification of the user and fully effective and reliable data detection of biomedical parameter of the user.

The present invention therefore relates to a system and method for detecting biomedical parameters from a subject seated on a toilet seat as defined in basic terms in the appended claims 1 and 19, respectively.

Additional preferred features of the invention are indicated in the dependent claims.

The system and method of the invention are particularly advantageous since all the sensors make use of just one seamless interface with the user, which does not require metallic electrodes, and enables the intrinsic identification of the user through an integrated system that collects the data using methods that do not require placement of the sensor arrangement on the subject.

As previously mentioned, user identification is a particularly critical feature, in the sense that sanitary facilities are used by a multitude of individuals, and mapping both the extracted features and the results of the analysis to the correct individual is of utmost importance for the validity of clinical decisions.

Another particularly advantageous feature of the present invention is that the sensory unit uses a virtual ground in the embodiments that require direct contact with the user, enabling the use of only two contact points with the subject specifically for Electrocardiographic (ECG) data acquisition.

Other features of the invention, implemented in preferred embodiments, are also particularly advantageous with respect to the known systems.

In particular, the toilet seat is specifically designed to promote a high level of hygiene in the use of the system, as well as the identification of the user by using signals collected through non-metallic sensors integrated in the toilet seat.

Basically, the invention is based on use of a system comprising a control unit, a sensory unit, and a power supply unit; the system as a whole is designed to perform the measure of body parameters by detecting and processing signals from the user body, and to display related information representing biomedical parameters of: electrocardiography (ECG), photoplethysmography (PPG), functional near infrared spectroscopy (fNIRS), phonocardiography (PCG), inertial measurement unit (IMU), radio interferometry (RI), optical fiber (OF), video and/or thermal imaging (VTI).

The system of the invention is configured to perform biometric recognition and/or biomedical monitoring of users of sanitation facilities in a continuous way, even though it can perform the same function momentarily, in the same way performed by other biomedical sensing devices.

### BRIEF DESCRIPTION OF THE DRAWINGS

Further characteristics and advantages of the present invention will become clear from the description of the following non-limitative embodiments, with reference to the figures in the accompanying drawings, in which:
- Figure 1 is a schematic view of a system for detecting biomedical parameters from a subject seated on a toilet seat, in accordance with a first embodiment of the invention, integrated in a toilet seat assembly for toilets or other sanitary facilities;

- Figure 2 is a block diagram showing the main components of the system of the invention;
- Figures 3 and 4 are a perspective view and a lateral view, respectively, of a sensor terminal for use in the system of the invention;
- Figures 5 and 6 are a perspective view and a lateral view, respectively, of a variation of the sensor terminal of Figures 3-4;
- Figure 7 is a perspective view of another variation of the sensor terminal of Figures 3-4;
- Figures 8 to 10 are respective schematic views of further embodiments of the system in accordance with the invention, integrated in a toilet seat assembly.

### BEST MODE FOR CARRYING OUT THE INVENTION

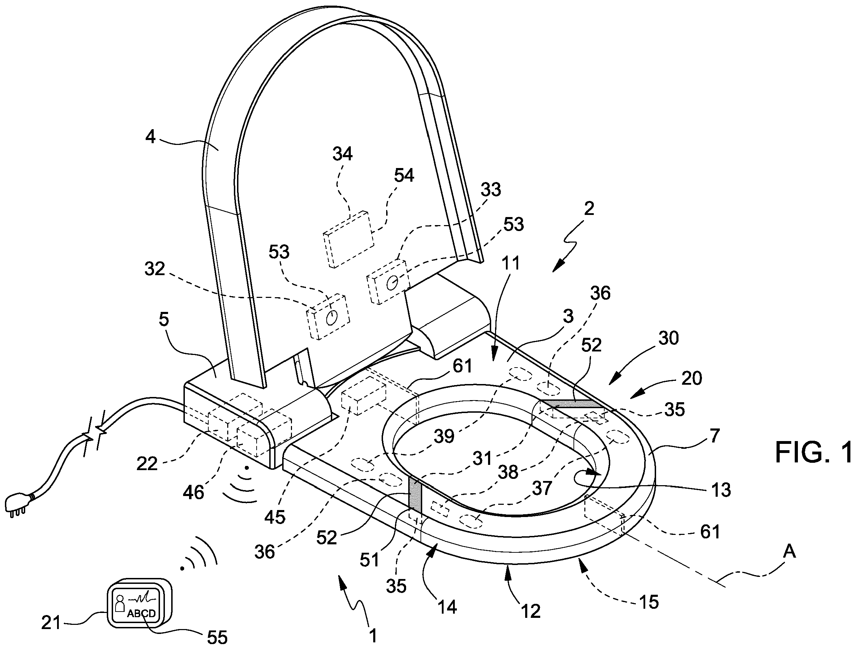

Figure 1 schematically shows a system 1 for detecting biomedical parameters from a subject (a person) seated on a toilet seat (i.e. a seat for a toilet or WC).

The system 1 is integrated in a toilet seat assembly 2 comprising a toilet seat 3, a lid 4, and a support 5 that can be fixed to a sanitary bowl (known and not shown) in any known manner (for example, by means of a pair of bolts or other fastening members) and is hinged to the seat 3 and to the lid 4, for example through respective rotation pins (not shown).

The seat 3 has a substantially annular body 7, which can take various forms, also based on the shape of the bowl on which it is to be mounted. In general, the body 7 extends along a central axis A and has a longitudinally elongated donut shape.

Optionally, the body 7 does not form a closed (complete) ring, but is frontally open, i.e. is provided with a radial through cut in a front area of the seat 3.

The body 7 extends between an upper surface 11 and a lower surface 12, opposite to each other; and has a radially inner peripheral edge 13 and a radially outer peripheral edge 14, facing to each other and extending between the upper surface 10 and the lower surface 12.

The body 7 can be a hollow body, in which the upper surface 11 and the edges 13, 14 delimit a substantially annular cavity 15 beneath the surface 11; or the body 7 can be a substantially solid body, provided with one or more cavities 15 formed in the body 7.

The system 1 is configured to perform biometric recognition and biomedical monitoring of users of sanitation facilities, in particular, users of the toilet seat assembly 2, preferably in a continuous way (even though the system 1 can perform the same functions also at predetermined instants, i.e. momentarily).

The system 1 comprises: a sensory unit 20, a control unit 21, and a power supply unit 22.

According to different embodiments of the invention, the sensory unit 20, the control unit 21, and the power supply unit 22 can be differently shaped and configured and variously arranged and positioned with respect to one another and to the components of the toilet seat assembly 2.

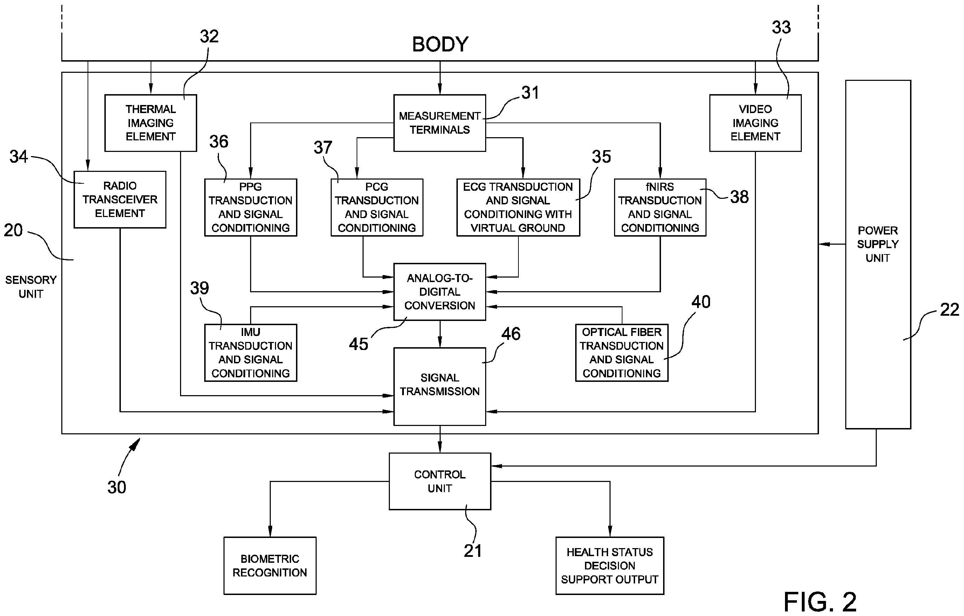

Some exemplary embodiments of the invention are described in details hereinbelow, while the general configuration of the system 1 is shown in Figure 2, which is a block diagram depicting the main components of the system 1, i.e. the sensory unit 20, the control unit 21, and the power supply unit 22.

With reference to both Figures 1 and 2, the sensory unit 20 is configured to detect signals from the user (seated on the toilet seat 3 in a normal position of use) and to process said signals in a form to be transmitted to the control unit 21.

In particular, the sensory unit 20 comprises one or more electronic sensor modules 30 capable of detecting signals from the user and selected (either individually or in any combination) from:
contact measurement module 31;
thermal imaging module 32;
video imaging module 33;
radio transceiver module 34;
ECG (electrocardiography) module 35;
PPG (photoplethysmography) module 36;
PCG (phonocardiography) module 37;
fNIRS (functional near infrared spectroscopy) module 38;
IMU (inertial measurement unit) module 39;
optical fiber module 40.

The sensor modules 30 are configured to detect signals (representative of body parameters) from the user, and/or to process said signals, in particular by transduction and signal conditioning, operating individually or together with other sensor modules 30.

If one or more sensor modules 30 are configured to provide analog signals, the system 1 additionally comprises an analog-to-digital conversion module 45, connected to the sensor modules 30 to convert analog signals provided by the sensor modules 30 to digital signals, if needed.

The system 1 also comprises a signal transmission module 46, configured to receive signals from the sensor modules 30 (possibly converted by the analog-to-digital conversion module 45) and transmit said signals to the control unit 21.

As already mentioned, the sensor modules 30 may include a number of different modules, configured to detect and process respective signals representative of different parameters of the user, and/or to process said signals.

Basically, the sensor modules 30 comprise respective electronic circuits possibly associated with sensors of different types. Depending on the operating principle of the sensors, the sensor modules 30 can be in direct contact with any part of the user's body or not.

The right and/or left legs (in particular, the respective thighs) are the preferred application points in the case where contact is needed, while the back of the trunk and buttocks are the preferred zones in the vicinity of which contactless sensor modules (not requiring direct contact with the user body) are placed, although these are merely illustrative examples.

In particular, the contact measurement module 31 comprises at least one sensor 51 defining a direct interface with the user skin for acquiring ECG, PPG, PCG, and fNIRS signals, which are then processed by the ECG module 35, the PPG module 36, the PCG module 37, the fNIRS module 38.

According to the invention, the sensor 51 is a contact sensor which is positioned, in use, in direct contact with a part of the user's body, as detailed hereinbelow.

The sensors 51 can be based on different operating principles: in particular, the sensors 51 of the contact measurement module 31 operates by detecting the electrical potential difference measured between two sensor terminals 52 in contact with the body (skin) of the user; in addition, the contact measurement module 31 can also include sensors capable of detecting changes in the electromagnetic field measured using a capacitive element, or capable of measuring mechanical actions of any type (such as piezoelectric, or pressure sensors, etc.), without necessarily requiring a direct contact with the user body.

The contact measurement module 31 can be either integrated in the sensory unit 20 (possibly together with other modules 30) or consist of an independent module, connected to the remaining modules 30 of the sensory unit 20 by wires or cables.

In particular, the contact measurement module 31 is connected to the ECG module 35, the PPG module 36, the PCG module 37, and the fNIRS module 38.

The thermal imaging module 32, the video imaging module 33, and the radio transceiver module 34 typically work in proximity to but not in direct contact with the skin of the user.

In particular, the thermal imaging module 32 and the video imaging module 33 include respective cameras or other similar imaging elements 53 configured to acquire images (thermal images or optical images respectively) from the user. The thermal imaging module 32 and the video imaging module 33 (with the respective imaging elements 53) are advantageously housed in the lid 4, in proximity of a lower edge thereof hinged to the support 5 and close to the toilet seat 3, or in another structure of the toilet seat assembly 2 or other associated installations.

The output of the thermal imaging module 32 and the video imaging module 33 allows a further enhancement of the method of the invention for biometric recognition and/or biomedical monitoring. In fact, image acquisition and processing can be used by the system 1 to capture footage from the body of the user and/or for analysis of substances excreted by the user.

The radio transceiver module 34 comprise one or more radio transceiver elements 54 configured to emit and/or receive radio waves in the GHz range, so as to detect mechanical displacements associated with the cardiovascular activity of the body. The radio transceiver elements 54 of the radio transceiver module 34 can be fitted on the toilet seat 3 or the lid 4.

The ECG module 35, the PPG module 36, the PCG module 37 and the fNIRS module 38 are all connected to the contact measurement module 31 to process data received therefrom.

Each of the ECG module 35, the PPG module 36, the PCG module 37 and the fNIRS module 38 comprise an electronic circuit configured to perform transduction and signal conditioning.

The electrical voltages associated with the cardiovascular system activity are measured by using the ECG module 35, which receives ECG signals from the contact measurement module 31, acquired by the pair of terminals 52 of the sensors 51, and provides to the transduction and conditioning with virtual ground of the ECG signals.

In particular, the ECG module 35 is configured to perform signal transduction and conditioning with virtual ground of ECG data.

In more details, the ECG module 35 is configured to perform the detection and conversion of physical parameters, detected by the contact measurement module 31, into signals and to filter and amplify said signals so that the signals can be treated as an electrical quantity. Preferably, the electronic circuit of the ECG module 35 includes an electromagnetic noise filter, compatible with the transmission of the signals through a wireless connection; and an amplifier. As a result, the electrical quantity is a clean and high definition representation of the physical quantity, free of external noise.

Preferably, the ECG module 35 is equipped with a band pass filter, particularly advantageous in the present invention by having a passing band between 0.5 and 40Hz, so as to eliminate the need for traditional notch filters, simplifying the physical construction of the module while allowing an adequate separation between the ECG signal and parasite signals such as motion artifacts, baseline wander, muscle signals, power line interference, among others.

Amplification occurs with a gain between 10 and 40000, allowing a significant increase in the definition of the collected signal (in the order of µV, mV or V), making the tenuous ECG signals more immune to external noise, and enabling a sufficient definition for the biometric recognition and/or biomedical monitoring method to operate.

According to the invention, the ECG module 35 is particularly advantageous insofar the sensors 51 of the contact measurement module 31 operate in direct contact with the user body: in fact, according to the invention, the ECG module 35 has an electronic circuit configured so as to generate a virtual ground, i.e. to provide a virtual reference voltage, enabling the use of only two measurement terminals and eliminating the need for the traditional third electrode that collects the real ground, indispensable for the acquisition of ECG signals by traditional methods.

The electronic circuit of the ECG module 35 provides a stable reference voltage potential to the signal conditioning circuit, without requiring a direct connection to reference potential; for example, the virtual ground can be implemented using a voltage divider, a rail splitter or any other analogous circuit. The PPG module 36 is configured to perform transduction and signal conditioning of PPG (photoplethysmography) data. In particular, the PPG module 36 is configured to perform the conversion and detection of physical parameters received from the contact measurement module 31 into signals, and to filter and amplify the resulting signals so that the signals can be treated as an electrical quantity. Preferably, also the module 36 of PCG transduction and signal conditioning includes an electromagnetic and mechanical noise filter, compatible with the vibrations transmitted to the contact measurement module 31 and/or with the transmission of the signals through a wireless connection, resulting in that the electrical quantity is a clean and high definition representation of the physical quantity, free of external noise.

The fNIRS module 38 is configured to measure optical processes associated with the cardiovascular activity of the body. According to the invention, the fNIRS module 38 is directly integrated in the toilet seat 3, in order to promote a contact or line-of-sight proximity with the skin of the user.

The fNIRS module 38 is configured to perform transduction and signal conditioning of fNIRS data. In particular, the fNIRS module 38 is configured to perform the conversion and detection of the physical quantity through the contact measurement module 31, filters the resulting signal, and amplifies it so that it can be treated as an electrical quantity. Also the module 38 preferably includes an electromagnetic and optical noise filter, compatible with the luminous interference transmitted to the contact measurement module 31 and/or with the transmission of the signals through a wireless connection. As a result of the operation of the module, the electrical quantity is a clean and high definition representation of the physical quantity, free of external noise.

The fNIRS module 38 includes at least one optical emitter and one optical receiver, although it can include more elements working at different luminous wavelengths to enable a more diverse observation of the vascular dynamics, as a way to obtain more reliable biometric recognition and/or results of biomedical monitoring.

Similarly to other modules, also the fNIRS module 38 has an electronic circuit configured to filter and amplify the signals, in order to produce a more suitable representation of the physical quantity. Also in this case the filter is preferably a band pass filter, particularly advantageous in the present invention, which, by having a high pass cutoff frequency of 0.5Hz and a selectable low pass cutoff frequency of between 5Hz and 1kHz, enables a wide dynamic range, simplifying the physical construction of the device while allowing an adequate separation between the underlying physiological processes. The amplification has a gain between 1 and 40000, allowing the increase in the definition of the collected signal, making the tenuous fNIRS signals more immune to external noise. The intensity of the emitted light can also be reduced or increased, in order to improve the definition of the tenuous fNIRS signals and make the output more immune to external noise. The overall design of this sensor enables a sufficient definition for the biometric recognition and/or biomedical monitoring method to operate.

The mechanical displacements associated with the cardiovascular activity of the body, propagated onto the toilet seat cover, are measured using the PCG module 37, the IMU module 39, and/or the optical fiber module 40. All said modules are advantageously integrated in the toilet seat 3 or another structure of the sanitary facilities and operates without needing a direct contact with the user (as indeed required for the signal acquisition by using traditional methods).

Each of the PCG module 37, IMU module 39, and optical fiber module 40 has an electronic circuit configured to perform the filtering and amplification of the respective signals, producing a more suitable representation of the physical quantity by filtering and amplifying.

Preferably, the electronic circuit comprises a band pass filter, particularly advantageous in the present invention which, by having a high pass cutoff frequency of 0.5 Hz and a selectable low pass cutoff frequency of between 50 Hz and 50 kHz, enables a wide dynamic range, simplifying the physical construction of the device while allowing an adequate separation between the PCG, IMU and/or optical fiber signal and parasite signals such as motion artifacts or baseline wander, among others.

The circuit also include an amplification circuit configured to provide an increase between 1 and 40000, allowing a significant increase in the definition of the collected signal, making the tenuous PCG, IMU and/or optical fiber signals more immune to external noise, and enabling a sufficient definition for the biometric recognition and/or biomedical monitoring method to operate.

Preferably, the PCG module 37, the IMU module 39, and/or the optical fiber module 40 are positioned close to the point of interface with the user (i.e. near the terminals 52), so significantly reducing the appearance of parasite signals.

The PCG module 37 and/or the IMU module 39 can be integrated in the contact measurement module 31.

The optical fiber module 40 can consist of one or more polymer optical fiber Bragg grating elements placed for example along the toilet seat 3 and/or the lid 4.

In more details, the IMU module 39 is configured to perform transduction and signal conditioning of IMU data. In particular, the IMU module 39 is configured to autonomously perform the conversion and detection of the physical quantity, to filter the resulting signal, and to amplify the signal so that it can be treated as an electrical quantity. Preferably, the module 39 of IMU transduction and signal conditioning includes an electromagnetic and mechanical noise filter, compatible with the vibrations transmitted to the contact measurement module 31 and/or with the transmission of the signals through a wireless connection. As a result of the operation of this module, the electrical quantity is a clean and high definition representation of the physical quantity, free of external noise.

The optical fiber module 40 is configured to perform transduction and signal conditioning of optical data. In particular, the module 40 is configured to perform the conversion and detection of the physical quantity autonomously, filter the resulting signal, and amplify it so that it can be treated as an electrical quantity. The module 40 preferably includes an electromagnetic and optical noise filter, compatible with the luminous interference transmitted to the contact measurement module 31 and/or with the transmission of the signals through a wireless connection. As a result of the operation of this module, the electrical quantity is a clean and high definition representation of the physical quantity, free of external noise.

The analog-to-digital conversion 45 module is configured to transform the electrical quantity, obtained through the ECG module 35, the PPG module 36, the PCG module 37, the fNIRS module 38, the IMU module 39, the optical fiber module 40, in an adequate digital representation manageable in the control unit 21.

The module 45 includes an electronic circuit that integrates a quantization element and an analog-to-digital converter. The quantization element is a component that maps the voltage or current to a set of bits (also known as resolution), which in the case of the present invention preferably ranges between 8 and 64 bits. The analog-to-digital converter is a component that, at regular and pre-defined time intervals, collects a sample, which is then quantized. In the case of the present invention, the frequency at which the samples are collected (sampling rate) can range between 250Hz and 16kHz, which in samples collected per unit of time corresponds respectively to 250 and 16000 samples per second.

The signal transmission module 46 is configured to transmit the digital representation generated by the analog-to-digital conversion module 45 to the control unit 21. The signal transmission module 46 and the control unit 21 may communicate with each other, for example, by a cabled or wireless connection, or by means of electrical tracks on a printed circuit board (for example, if the modules are integrated).

The signal transmission module 46 can operate by using several methods. Preferably, the module 46 uses a wireless channel based on existing protocols such as Bluetooth, WiFi, ZigBee, Narrow Band IoT, SigFox, LoRa, ANT, GPRS, and/or 3G/4G/5G, although other protocols can also be admissible.

Alternatively, the transmission can also be performed in a cabled manner, which in the present invention is also advantageous since it can be performed through more conventional interfaces, such as USB, COM/RS232, GPIO pins, tracks in a printed circuit board, direct connection to the Rx/Tx pins on a micro controller, and others.

The data can be transmitted from any one of the analog-to-digital conversion module 45, the thermal imaging element 32, the video imaging element 33, and/or from the radio transceiver module 34, as plain raw data, which is the general approach. Nevertheless, the data can also be transmitted as a transformed version of the raw data, computed using non-reversible functions (e.g. dissimilarity metrics) while preserving the informative content of the original data to the best extent possible.

The control unit 21 can be any type of electronic device suitable for digital processing and presentation of signals and data.

The control unit 21 can be a dedicated control unit, for example a microcontroller unit, either integrated in any part of the toilet seat assembly 2, for example in the toilet seat 3, or located in a remote position; the control unit 21 can also be the same central control unit of the toilet or other sanitation facility in which the system 1 is integrated; the control unit 21 can also be integrated in a portable electronic device such as a tablet computer, a mobile phone, etc.

In any case, the control unit 21 is configured to process data received from the sensory unit 20 and collected in real time by the sensory unit 20 from the user, and to extract from said data information representative of the conditions of the user so as to perform the biometric recognition of the user and evaluate the health status of the user.

Preferably, the control unit 21 is associated with a display unit 55 configured to provide a graphical presentation and/or representation of the signals processed by the control unit 21, of the result of the recognition process, and/or of the result of the analysis of the signals. For example, the display unit 55 can be the screen of the dedicated electronic device in which the control unit 21 is integrated, or the screen of a smartphone or tablet computer or other electronic device, the screen of the central console of the toilet, or any other similar device.

Operation of the control unit 21, implementing the method of the invention, is described in details hereinbelow.

The power supply unit 22 can be any electric and/or electronic equipment, with galvanic isolation, suitable for providing an input voltage with which the system 1 can operate. For example, the power supply unit 22 can include one or more of: a connection to the mains; a battery (such as a Lithium-Polymer battery) or another energy storage device; a wireless receiver capable of receiving energy from a dedicated wireless emission source; a hydraulic generator installed in the toilet (or in another structure where water flows); a receiver capable of receiving energy from WiFi or other radio frequency signals emitted from an external emitter; a solar panel installed on the toilet or in another location in the sanitary facility; etc.

In use, the system 1 operates, implementing the method of the invention, as follows.

The sensor modules 30 of the sensory unit 20 detect respective signals from the user.

The signals detected and measured by the contact measurement module 31 are transmitted to one or more modules selected from: the ECG module 35, which performs transduction and signal conditioning with virtual ground and filtering and amplification of the ECG signals; the PPG module 36, which performs transduction and signal conditioning and filtering and amplification of the PPG signals; the PCG module 37, which performs transduction and signal conditioning and filtering and amplification of the PCG signals; the fNIRS module 38 which performs transduction and signal conditioning and filtering and amplification of the fNIRS signals.

If needed, all the above mentioned modules transmit data to the analog-to-digital conversion module 45 for the conversion into digital signals.

Also the signals from the IMU module 39, the optical fiber module 40, the thermal imaging element 32, the video imaging element 33, and the radio transceiver element 34, possibly converted in the analog-to-digital conversion module 45 if needed, are sent to the transmission module 46 and transmitted to the control unit 21.

The control unit 21 processes the signals received from the sensory unit 20 and produces a result (decision) about the biometric recognition of the user and/or about the health status and biomedical monitoring of the user.

The result produced by the control unit 21 is displayed by the display unit 55.

The control unit 21 is configured so as to implement a method that adjusts the way in which it operates, enabling the extraction of representative information from the signals collected from the user, the corresponding biometric recognition, the result of the biomedical assessment, and/or the production of a technical effect according to the produced biometric recognition and/or health status decision support output.

The process is preferably performed in a continuous way, that is ensuring that the biometric recognition and/or biomedical monitoring of users of sanitation facilities occurs uninterruptedly while the system 1 is being used, although it can also be performed in a momentary way.

The control unit 21 is also configured so as to implement a method for customization of its settings as a result of the biometric recognition of the user. In particular, the control unit 21, after identifying the user on the basis of the data received from one or more sensor modules 30, for example on the basis of ECG data processed by the ECG module 35, then commands activation of further modules sensors 30, to integrate customized biomedical analysis for the specific identified user.

The method implemented by the control unit 21 is particularly advantageous in that the method provides not only the digital representation of parameters produced by the modules 30; but it is also capable of extracting from the sensory unit 20 relevant information about the biometric recognition and/or biomedical monitoring of the user, even using signals that are collected at the thighs, which, for all the constituents of the sensory unit 20, are not the conventional operating locations.

In order to carry out the biometric recognition of the user, the control unit 21 includes (or has access to) a memory where a database of registered users is recorded. The database is initially compiled, for example, by using one or a combination of the modules 30 that constitute the sensory unit 20. The control unit 21 either integrates a database of known users, or communicates with a central server that stores that information remotely.

The control unit 21, in order to provide the biometric recognition of the user, receives data from the sensory unit 20, and process and compare the data with the stored data so as to identify the user.

The biometric recognition method used in the present invention is particularly advantageous given that it can produce a decision on the identification or authentication of the user in a continuous mode, that is, guaranteeing that the biometric recognition occurs uninterruptedly during the time in which the user interacts with the device, or just upon request, that is, in a momentary way, where the recognition is optionally effected and repeated at predetermined time intervals, spaced by several minutes, hours, or days. The method applies a set of machine learning and/or artificial intelligence methods, which use one or more of the signals collected by the sensory unit 20, in raw form or as an alternative representation generated from the representative information extracted from them, and that matches the resulting information with the patterns previously stored in the enrolled users database.

The recognition process comprises, in sequence, a pre-processing step, in which the signal received from the sensory unit 20 is pre-processed to provide a pre-processed signal; an extraction step, in which data representative of the user are extracted from the pre-processed signal; and a final classification step, in which a decision is produced identifying the user.

In the pre-processing step, an additional digital filtering step is implemented which complements those performed in the sensory unit 20.

The extraction step automatically learns a representation (e.g. by means of an artificial neural network) or performs the segmentation of the collected signals (or, in alternative, just a few components of the same). Representative information about the complexes can also be extracted for each user, such as latencies, amplitudes, spectral density and others; furthermore, the average of several signal waveforms or of the extracted information can also be used. These are also particularly advantageous properties of the present invention.

In the classification step, the representative information is compared with the data in the database, to determine or validate the identity of the user by using an Artificial Neural Network (ANN), or, alternatively, a nearest neighbor (k-NN) approach with Euclidean distance as similarity metric, or Support Vector Machines (SVM). Still, other classification methods are admissible in the context of the present invention.

Finally, a decision about the biometric recognition of the user and/or about the health status support is produced in the control unit 21.

As already mentioned, the control unit 21 preferably provides the results/decisions by the display unit 55, in particular by means of the on-board display or a projection device, a mobile app, a cloud-based portal, an online database, just to name a few examples, although other analogous methods are admissible.

The result produced by the control unit 21 can have different technical effects, which include but are not limited to: identification of the user of the toilet seat, verification of the identity of the user of the toilet seat, presentation of decisions and results of the biomedical monitoring analysis to the user, customization of settings according to the perceived user, issue of notifications pertaining the health status of the user.

For example, but not necessarily, the method implemented in the control unit 21 uses, as input data received from the sensory unit 20, data, in raw or transformed form, selected from: ECG waveform, PCG waveform, fNIRS waveform (in the different luminous wavelengths), IMU waveform, optical fiber waveform, radio transceiver absorption waveform, thermal imaging footage, and/or optical video imaging footage. Based on these data, the method can output, amongst other parameters: user identity, usage frequency, arrhythmia detection, optical-optical pulse transit time, electrical-optical pulse transit time, force exerted in the toilet seat, pressure mapping, user weight, respiration data, stool analysis, approximated fecal weight, approximated fecal consistency, urine analysis, atrial fibrillation, blood oxygenation, blood flow, approximated blood pressure, body fat, heart rate, heart rate variability, temperature, and/or posture.

The device is designed for continuous operation and, as such, it can be used to assess the user's health status as an extension of the normal everyday practices that the user already performs within sanitary facilities.

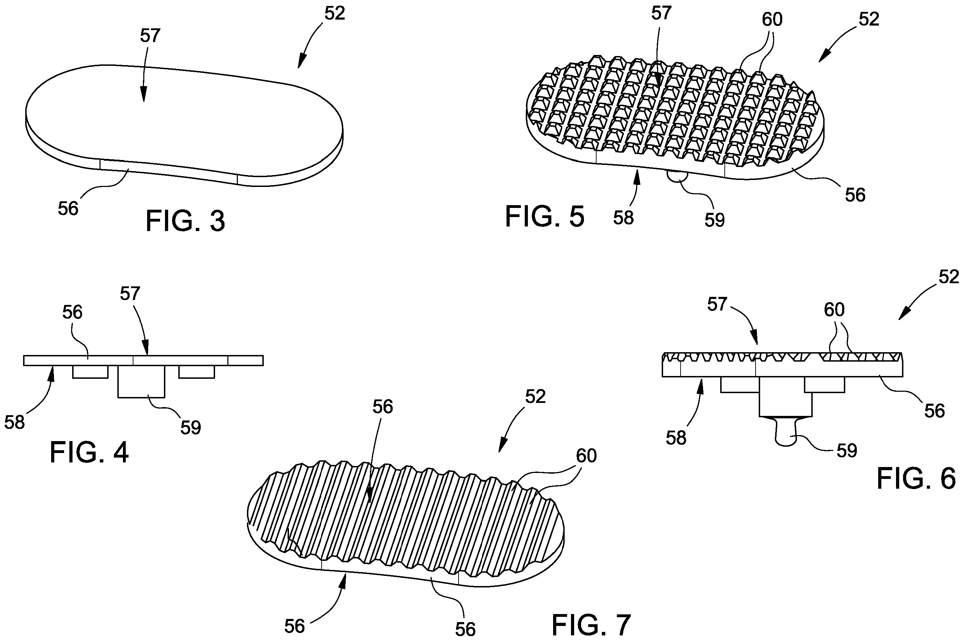

Figures 3 to 7 show preferred embodiments of the sensor terminals 52 of the contact measurement module 31.

With reference to Figures 3-4, each terminal 52 comprises a substantially flat pad 56 (which can have different shape and size), having a conductive top surface 57 for contacting the user body and a bottom surface 58, opposite to the top surface 57, provided with connectors 59 for electrical connection. The terminals 52 are housed in the toilet seat 3 and are positioned so as the top surface 57 is substantially flush with the upper surface 11 of the toilet seat 3. The terminals 52 are positioned so as to contact, when the user is seated on the toilet seat 3 in a normal position of use, the left and right thighs respectively of the user.

Advantageously, the conductive top surface 57 of each terminal 52 covers the whole respective section of the toilet seat 3 (right or left sections respectively), extending substantially between the radially inner peripheral edge 13 and the radially outer peripheral edge 14 of the toilet seat 3.

According to a preferred aspect of the invention, the terminals 52 or at least a portion thereof comprising the top surface 57 (for example, the pad 56) are advantageously made of non-metallic materials, such as conductive coatings, conductive ceramics, conductive polymer materials (plastic), indium tin oxide (ITO), or other materials that are functionalized to eliminate the need to use any kind of explicitly metallic element that modifies the typical appearance of a toilet seat and/or the need for conductive gel or paste.

For example, in a preferred embodiment, the sensor 51 has terminals 52 consisting of a ceramic core functionalized with an outer ITO coating, so as there are no metal parts (pads) and there is no need for using conductive gel or paste for a good interface with the user skin.

According to the invention, only two terminals 52 are needed in order to detect ECG signals from the user body.

In fact, the terminals 52 allow the measurement of ECG signals between the left and right thighs.

The contact measurement module 31 is particularly advantageous in that, in addition to the measurement function, the terminals 52 which are in contact with the body of the user can also perform additional operations: for example, the terminals 52 can be configured so as to generate heat and/or electrical stimulation, so as to act as heating pads and/or as electrical stimulation pads thus providing an actuation function on the user body.

Moreover, as shown in Figures 5 to 7, in some preferred embodiments the terminals 52 are provided with projections 60, protruding from the top surface 57 of the pad 56 to increase the contact between the terminals 52 (and thus the contact measurement module 31) and the skin of the user, as a way of overcoming natural barriers of the body, such as hair (in fact, body hair constitutes a barrier to the conduction of electrical signals).

The projections 60 can be variously shaped and arranged according to different patterns. For example, in the embodiment of Figures 5-6 the pad 56 is provided with a grid of projections 60 having a truncated sphere, truncated cone or truncated pyramid shape, arranged in rows. In the embodiment of Figure 7 the pad 56 is provided with a series of transversal projections 60 in the form of ribs parallel to one another and defining a series of alternate protruding concavities and/or convexities.

The physical position of the ECG module 35 with respect to the contact measurement module 31 and the terminals 52 is also particularly advantageous according to the invention. Although several other configurations are admissible in the context of the invention, the ECG module 35 is more advantageously arranged in close proximity of the point of interface with the user (i.e. in close proximity of the terminals 52) so as to greatly minimize the appearance of parasite signals. In traditional signal acquisition methods, ECG modules are usually placed far away from the point of interface with the user, making the cabled connection work as an antenna that captures several surrounding noise sources.

If the contact measurement module 31 comprises only two terminals 52, the two terminals 52 are positioned on opposite sides of the toilet seat 3, i.e. on opposite sides of the central axis A, in positions corresponding to the left and right thighs of the user seated on the toilet seat 3. The two terminals 52 are separated by insulating elements 61 positioned between the terminals 52 in the toilet seat 3, for example along the central axis A, to ensure that there is no electrical path between the two terminals 52.

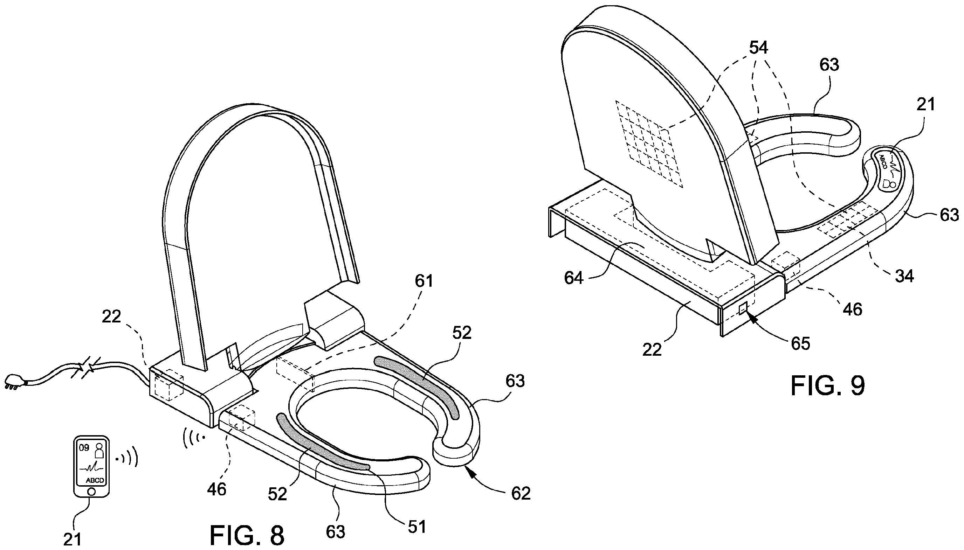

If the toilet seat 3, as shown for example in Figure 8, has a front opening 62 that divides two substantially parallel arms 63 of the annular body 7, only one insulating element 61 is needed at the rear portion of the annular body 7, opposite to the front opening 62, where the arms 63 are joined.

If more than two terminals 52 are used, each terminal 52 needs to be insulated from other contiguous terminals 52. The use of more terminals 52 results in a larger area available for contact with the user and favors the acquisition of very high definition and low noise signals, without affecting the ability to adequately clean neither the surface nor the natural aesthetics of the toilet seat.

It remains understood that the sensory unit 20, the control unit 21 and the power supply unit 22, as well as the sensor modules 30, can be variously arranged with respect to one another.

In a preferred embodiment, the sensory unit 20 and the control unit 21 are integrated in one another.

In other embodiments, however, the sensory unit 20 and the control unit 21 are separated from one another and arranged in different positions; the signal transmission module 46 ensures communication between the sensory unit 20 and the control unit 21.

For example, in an embodiment the sensory unit 20 and the transmission module 46 are housed in the toilet seat 3 or in the lid 4, while the control unit 21 is in a remote position, being for instance the toilet seat central console or a dedicated device.

It remains understood, however, that all the previously described examples do not limit the scope of the invention as defined by the appended claims. In particular, the control unit 21 can be defined by a plurality of different devices, not only a dedicated hardware device, specifically designed as a component of the system 1, but also by an existing hardware device, such as a portable electronic device (smartphone, smart watch, tablet, notebook, portable computer, etc.) or any kind of computer or processing unit.

In the embodiment of Figure 8, the control unit 21 is integrated in a smartphone. The contact measurement module 31 comprises two terminals 52 arranged on opposite sides of the toilet seat 3, i.e. of the central axis A. In this case, the terminals 52 have a longitudinally elongated shape along respective lateral portions of the annular body 7 of the toilet seat 3.

The terminals 52 can have the features previously described.

In the embodiment of Figure 9, the control unit 21 is housed in the toilet seat 3, in particular in a front portion of one of the arms 63; and the power supply unit 22 comprises a battery 64 housed in the support 5 and having a connector 65 (e.g. a USB connector) for recharging the battery 64.

In this embodiment, the radio transceiver module 34 comprises a plurality of radiotransceiver elements 54 arranged in different positions on the toilet seat 3 and/or the lid 4.

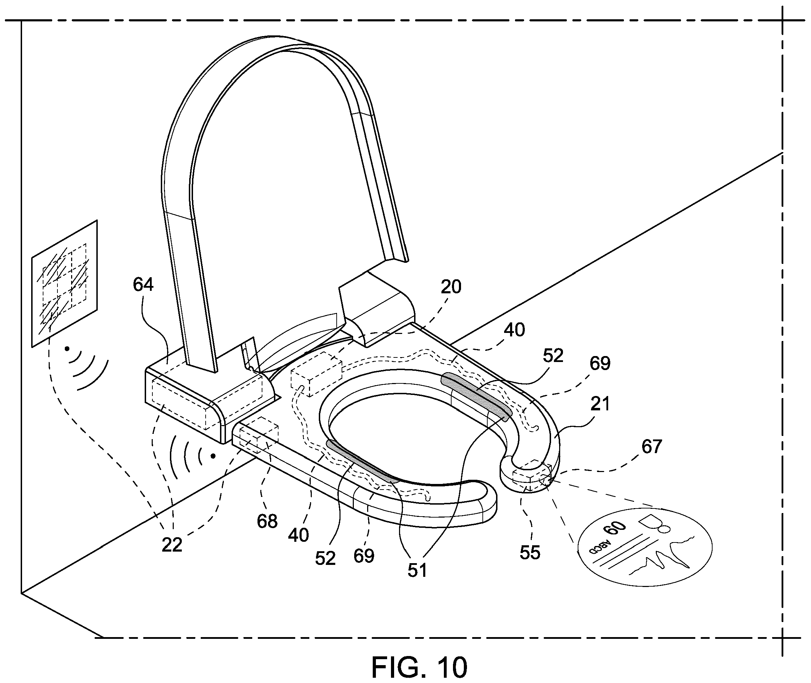

In the embodiment of Figure 10, the control unit 21 is associated with a display unit 55 comprising a projection device 67 which is configured to project information elaborated by the control unit 21 on any kind of screen, for example directly on the floor in front of the toilet seat assembly 2.

In this embodiment, the power supply unit 22 additionally includes both a battery 64 and a wireless and/or WiFi receiver 68 capable of receiving energy from an external dedicated wireless emission source or from an external WiFi or other radio frequency signals emitter.

The terminals 52 of the contact measurement module 31 have a longitudinally elongated shape along respective lateral portions of the annular body 7 of the toilet seat 3; the top surface 57 of each terminal 52 extends to cover both a portion of the upper surface 11 of the toilet seat 3, and a portion of the inner peripheral edge 13 of the toilet seat 3.

The optical fiber module 40 comprises optical fiber elements 69, for example polymer optical fiber Bragg gratings, placed on the toilet seat 3 and in particular along opposite lateral portions (on opposite sides of the central axis A) of the toilet seat 3.

Of course, all the embodiments described above and any individual feature or component thereof can be combined with one another.

Finally, it is understood that further modifications and variants may be made to the method and system described and illustrated herein without departing from the scope of the appended claims.

## Claims

1. A system for detecting biomedical parameters from a subject seated on a toilet seat, comprising: a toilet seat assembly (2) comprising at least a toilet seat (3); a sensory unit (20) comprising one or more electronic sensor modules (30) configured to detect data from a user seated on the toilet seat (3) in a normal position of use; a control unit (21) connected to the sensory unit (20) to receive data therefrom; and a power supply unit (22) to supply power to the sensory unit (20) and the control unit (21);
wherein the sensor modules (30) comprise at least a contact measurement module (31) having at least one sensor (51) defining a direct interface with the user body for acquiring ECG signals from the user; and an ECG (electrocardiography) module (35), configured to process ECG signals received from the contact measurement module (31);
and wherein the sensor (51) of the contact measurement module (31) comprises a pair of sensor terminals (52) positioned on the toilet seat (3) so as to be in direct contact with respective thighs of the user seated on the toilet seat (3) in a normal position of use; the sensor (51) being configured to detect an electrical potential difference measured between said two terminals (52) in contact with the body of the user;
**characterized in that** the contact measurement module (31) comprises only two terminals (52), positioned on opposite sides of the toilet seat (3) and of a central axis (A) thereof, in positions corresponding to the left and right thighs of the user seated on the toilet seat (3); the two terminals (52) being separated by one or more insulating elements (61) positioned between the terminals (52) in the toilet seat (3);
and **in that** the ECG module (35) is configured to perform signal transduction and conditioning with virtual ground of the ECG signals received from the contact measurement module (31), the ECG module (35) having an electronic circuit configured so as to generate a virtual ground signal, i.e. to provide a virtual reference voltage, enabling the use of only two measurement terminals for the acquisition of ECG signals.

2. A system according to claim 1, wherein the ECG module (35) has an electronic circuit configured to perform detection and conversion of physical parameters, acquired by the contact measurement module (31), into signals and to filter and amplify said signals so that said signals can be treated as an electrical quantity to be transmitted to the control unit (21).

3. A system according to any one of the preceding claims, wherein the ECG module (35) is arranged on the toilet seat (3) in proximity of the terminals (52) of the contact measurement module (31) defining the interface with the user.

4. A system according to any one of the preceding claims, wherein the two terminals (52) are separated by one or more insulating elements (61) positioned between the terminals (52) in the toilet seat (3) along the central axis (A).

5. A system according to any one of the preceding claims, wherein the electronic circuit of the ECG module (35) includes a band pass filter having a passing band between 0.5 and 40Hz; and an amplifier, providing an amplification with a gain between 10 and 40000.

6. A system according to any one of the preceding claims, wherein the sensory unit (20) comprises one or more electronic sensor modules (30) selected from a PPG (photoplethysmography) module (36), a PCG (phonocardiography) module (37), a fNIRS (functional near infrared spectroscopy) module (38); and the contact measurement module (31) is configured to acquire, in addition to ECG signals, one or more of PPG, PCG, and fNIRS signals, which are then processed by the ECG module (35), the PPG module (36), the PCG module (37), the fNIRS module (38) respectively; and wherein the ECG module (35), the PPG module (36), the PCG module (37) and the fNIRS module (38) are all connected to the contact measurement module (31) to process data received therefrom; each of the ECG module (35), the PPG module (36), the PCG module (37) and the fNIRS module (38) comprising an electronic circuit configured to perform transduction and signal conditioning.

7. A system according to any one of the preceding claims, wherein the sensory unit (20) comprises a thermal imaging module (32) and/or a video imaging module (33) including respective cameras or other imaging elements (53) configured to acquire images, thermal images or optical images respectively, from the user; the thermal imaging module (32) and the video imaging module (33) with the respective imaging elements (53) being housed in the lid (4) of the toilet seat assembly (2).

8. A system according to any one of the preceding claims, wherein the sensory unit (20) comprises a radio transceiver module (34) comprising one or more radio transceiver elements (54) configured to emit and/or receive radio waves in the GHz range, so as to detect mechanical displacements associated with the cardiovascular activity of the user body.

9. A system according to any one of the preceding claims, comprising a signal transmission module (46), configured to receive data from the sensor modules (30), either directly or converted by an analog-to-digital conversion module (45), and transmit said data to the control unit (21).

10. A system according to any one of the preceding claims, wherein the control unit (21) is a dedicated control unit, for example a microcontroller unit, either integrated in any part of the toilet seat assembly (2), for example in the toilet seat (3), or located in a remote position; or the control unit (21) is integrated in another central control unit of a toilet or other sanitation facility in which the system (1) is integrated; or the control unit (1) is integrated in a portable electronic device such as a tablet computer, a mobile phone, etc.

11. A system according to any one of the preceding claims, wherein the control unit (21) is configured to process data received from the sensory unit (20) and collected in real time by the sensory unit (20) from the user, and to extract from said data information representative of the conditions of the user so as to perform the biometric recognition of the user and evaluate the health status of the user.

12. A system according to any one of the preceding claims, wherein the control unit (21) is configured to process data from the ECG module (35) so as to provide a biometric recognition of the user by comparing said data received from the ECG module (35) with a database of registered users.

13. A system according to any one of the preceding claims, wherein the control unit (21) is associated with a display unit (55) configured to provide a graphical presentation and representation of the signals processed by the control unit (21), of the result of the recognition process, and/or of the result of the analysis of the signals.

14. A system according to any one of the preceding claims, wherein the power supply unit (22) includes one or more of: a connection to the mains; a battery or another energy storage device; a wireless receiver capable of receiving energy from a dedicated wireless emission source; a hydraulic generator installed in the toilet or in another structure where water flows; a receiver capable of receiving energy from WiFi or other radio frequency signals emitted from an external emitter; a solar panel.

15. A system according to any one of the preceding claims, wherein each terminal (52) of the contact measurement module (31) has a conductive top surface (57) for contacting the user body and positioned so as to be substantially flush with an upper surface (11) of the toilet seat (3); the pair of terminals (52) being positioned so as to contact, when the user is seated on the toilet seat (3) in a normal position of use, the left and right thighs respectively of the user; and wherein the terminals (52) or at least a portion thereof comprising the top surface (57) are made of non-metallic materials selected from conductive coatings, conductive ceramics, conductive polymer materials, indium tin oxide (ITO).

16. A system according to claim 15, wherein the terminals (52) are provided with projections (60), protruding from the top surface (57) to increase the contact between the terminals (52), and thus the contact measurement module (31), and the skin of the user.

17. A method for detecting biomedical parameters from a subject seated on a toilet seat, comprising the steps of:
- providing a toilet seat assembly (2), comprising at least a toilet seat (3), with a sensory unit (20) comprising one or more electronic sensor modules (30); the sensor modules (30) comprising at least a contact measurement module (31) having at least one sensor (51) defining a direct interface with the user body for acquiring ECG signals from the user and comprising a pair of terminals (52) positioned on the toilet seat (3) so as to be in direct contact with respective thighs of the user seated on the toilet seat (3) in a normal position of use; wherein the contact measurement module (31) comprises only two terminals (52), positioned on opposite sides of the toilet seat (3) and of a central axis (A) thereof, in positions corresponding to the left and right thighs of the user seated on the toilet seat (3); the two terminals (52) being separated by one or more insulating elements (61) positioned between the terminals (52) in the toilet seat (3);
- detecting an electrical potential difference measured between said two terminals (52) of the contact measurement module (31); and generating a virtual ground signal, representative of a virtual reference voltage, enabling the use of only two measurement terminals for the acquisition of ECG signals;
- processing the signals detected by the contact measurement module (31) to provide a result comprising a biometric identification of the user and/or a representation of body parameters of the user;
- displaying the result.

18. A method according to claim 17, wherein the biometric identification of the user is performed in a continuous way, by continuously acquiring signals from the user and processing said signals along a period of time in which the user is seated on the toilet seat (2).

19. A method according to claim 17 or 18, comprising a step of customizing the information provided as a result of the method, by automatically adjusting the operation of the method according to the identity of the user as recognized by the method.

20. A method according to one of claims 17 to 19, wherein the biometric identification of the user is obtained by the steps of: processing data from the sensory unit (20) and comparing said data with data stored in a database of known users, so as to identify a current user.

21. A method according to one of claims 17 to 20, wherein the biometric identification of the user comprises, in sequence, a pre-processing step, in which the signal received from the sensory unit (20) is pre-processed to provide a pre-processed signal; an extraction step, in which data representative of the user are extracted from the pre-processed signal; and a final classification step, in which a decision is produced identifying the user.

22. A method according to one of claims 17 to 21, wherein the step of displaying the result comprises displaying one or more of: identification of the user; verification of the identity of the user; presentation of decision and results of the biomedical monitoring analysis; customization of settings to the user; notifications pertaining the health status of the user.

23. A method according to one of claims 17 to 22, comprising a step of acquiring and processing one or more of:
thermal images and/or video images of at least a part of the user body;
mechanical displacements associated with the cardiovascular activity of the body by detecting radio waves in the GHz range;
PPG (photoplethysmography) data;
PCG (phonocardiography) data;
fNIRS (functional near infrared spectroscopy) data;
IMU (inertial measurement unit) data;
optical fiber data.

## Patentansprüche

1. System zum Erfassen biomedizinischer Parameter von einem auf einem Toilettensitz sitzenden Subjekt, umfassend: eine Toilettensitzanordnung (2), die zumindest einen Toilettensitz (3) umfasst; eine Sensoreinheit (20), die ein oder mehrere elektronische Sensormodule (30) umfasst, die eingerichtet sind, Daten von einem Benutzer, der auf dem Toilettensitz
(3) in einer normalen Benutzungsposition sitzt, zu detektieren; eine Steuereinheit (21), die mit der Sensoreinheit (20) verbunden ist, um Daten von dieser zu empfangen; und eine Stromversorgungseinheit (22), um die Sensoreinheit (20) und die Steuereinheit (21) mit Strom zu versorgen;
wobei die Sensormodule (30) zumindest ein Kontaktmessmodul (31) mit zumindest einem Sensor (51) umfassen, der eine direkte Schnittstelle mit dem Körper des Benutzers definiert, um EKG-Signale von dem Benutzer zu erfassen; und ein EKG (Elektrokardiographie)-Modul (35), das eingerichtet ist, vom Kontaktmessmodul (31) empfangene EKG-Signale zu verarbeiten;
und wobei der Sensor (51) des Kontaktmessmoduls ein Paar Sensoranschlüsse (52) umfasst, die auf dem Toilettensitz (3) derart positioniert sind, um in direktem Kontakt mit den jeweiligen Oberschenkeln des auf dem Toilettensitz (3) in einer normalen Benutzungsposition sitzenden Benutzers zu stehen; wobei der Sensor (51) eingerichtet ist, um eine zwischen den beiden Anschlüssen (52) in Kontakt mit dem Körper des Benutzers gemessene elektrische Potentialdifferenz zu detektieren;
**dadurch gekennzeichnet, dass** das Kontaktmessmodul (31) nur zwei Anschlüsse (52) umfasst, die auf gegenüberliegenden Seiten des Toilettensitzes (3) und einer Mittelachse (A) desselben in Positionen angeordnet sind, die den linken und rechten Oberschenkeln des auf dem Toilettensitz (3) sitzenden Benutzers entsprechen; wobei die beiden Anschlüsse (52)
durch ein oder mehrere Isolierelemente (61) getrennt sind, die zwischen den Anschlüssen (52) im Toilettensitz (3) angeordnet sind;
und dadurch, dass das EKG-Modul (35) eingerichtet ist, eine Signaltransduktion und - konditionierung mit virtueller Masse der von dem Kontaktmessmodul (31) empfangenen EKG-Signale durchzuführen, wobei das EKG-Modul (35) eine elektronische Schaltung aufweist, die eingerichtet ist, ein virtuelles Massesignal zu erzeugen, d.h. eine virtuelle Referenzspannung bereitzustellen, wodurch die Verwendung von nur zwei Messanschlüssen für die Erfassung von EKG-Signalen ermöglicht wird.

2. System gemäß Anspruch 1, wobei das EKG-Modul (35) eine elektronische Schaltung aufweist, die eingerichtet ist, die Detektierung und Umwandlung von physikalischen Parametern, die von dem Kontaktmessmodul (31) erfasst werden, in Signale durchzuführen und die Signale derart zu filtern und zu verstärken, dass die Signale als eine elektrische Größe behandelt werden können, die an die Steuereinheit (21) übertragen wird.

3. System gemäß einem der vorhergehenden Ansprüche, wobei das EKG-Modul (35) auf dem Toilettensitz (3) in der Nähe der Anschlüsse (52) des Kontaktmessmoduls (31) angeordnet ist, die die Schnittstelle mit dem Benutzer definieren.

4. System gemäß einem der vorhergehenden Ansprüche, wobei die beiden Anschlüsse (52) durch ein oder mehrere Isolierelemente (61) getrennt sind, die zwischen den Anschlüssen (52) im Toilettensitz (3) entlang der Mittelachse (A) angeordnet sind.

5. System gemäß einem der vorhergehenden Ansprüche, wobei die elektronische Schaltung des EKG-Moduls (35) einen Bandpassfilter mit einem Durchlassbereich zwischen 0,5 und 40 Hz; und einen Verstärker mit einer Verstärkung zwischen 10 und 40000 aufweist.

6. System gemäß einem der vorhergehenden Ansprüche, wobei die Sensoreinheit (20) ein oder mehrere elektronische Sensormodule (30) umfasst, die ausgewählt sind aus einem PPG (Photoplethysmographie)-Modul (36), einem PCG (Phonokardiographie)-Modul (37), einem fNIRS (funktionelle Nahinfrarotspektroskopie)-Modul (38); und das Kontaktmessmodul (31) eingerichtet ist, zusätzlich zu den EKG-Signalen eines oder mehrere der PPG-, PCG- und fNIRS-Signale zu erfassen, die dann von dem EKG-Modul (35), dem PPG-Modul (36), dem PCG-Modul (37) bzw. dem fNIRS-Modul (38) verarbeitet werden; und wobei alle, das EKG-Modul (35), das PPG-Modul (36), das PCG-Modul (37) und das fNIRS-Modul (38) mit dem Kontaktmessmodul (31) verbunden sind, um davon empfangene Daten zu verarbeiten; wobei das EKG-Modul (35), das PPG-Modul (36), das PCG-Modul (37) und das fNIRS-Modul (38) jeweils eine elektronische Schaltung umfassen, die eingerichtet ist, eine Transduktion und Signalkonditionierung durchzuführen.

7. System gemäß einem der vorhergehenden Ansprüche, wobei die Sensoreinheit (20) ein Wärmebildmodul und/oder ein Videobildmodul (33) mit entsprechenden Kameras oder anderen Bildgebungselementen (53) umfasst, die eingerichtet sind, Bilder, Wärmebilder bzw. optische Bilder, vom Benutzer zu erfassen; wobei das Wärmebildmodul (32) und das Videobildmodul (33) mit den entsprechenden Bildelementen (53) im Deckel (4) der Toilettensitzanordnung (2) untergebracht sind.

8. System gemäß einem der vorhergehenden Ansprüche, wobei die Sensoreinheit (20) ein Funk-Transceiver-Modul (34) umfasst, das ein oder mehrere Funk-Transceiver-Elemente (54) umfasst, die eingerichtet sind, Funkwellen im GHz-Bereich derart auszusenden und/oder zu empfangen, um mechanische Verschiebungen zu detektieren, die mit der kardiovaskulären Aktivität des Körpers des Benutzers assoziiert werden.

9. System gemäß einem der vorhergehenden Ansprüche, das ein Signalübertragungsmodul (46) umfasst, das eingerichtet ist, Daten von den Sensormodulen (30) entweder direkt oder durch ein Analog-Digital-Wandlermodul (45) umgewandelt zu empfangen, und die Daten an die Steuereinheit (21) zu übertragen.

10. System gemäß einem der vorhergehenden Ansprüche, wobei die Steuereinheit (21) eine spezielle Steuereinheit ist, z.B. eine Mikrocontrollereinheit, die entweder in einem beliebigen Teil der Toilettensitzanordnung (2), z.B. im Toilettensitz (3), integriert ist oder an einer entfernten Position angeordnet ist; oder die Steuereinheit (21) ist in eine andere zentrale Steuereinheit einer Toilette oder einer anderen Sanitäreinrichtung integriert, in die das System (1) integriert ist; oder die Steuereinheit (1) ist in ein tragbares elektronisches Gerät, wie z.B. einen Tablet-Computer, ein Mobiltelefon usw., integriert.

11. System gemäß einem der vorhergehenden Ansprüche, wobei die Steuereinheit (21) eingerichtet ist, Daten zu verarbeiten, die von der Sensoreinheit (20) empfangen und in Echtzeit von der Sensoreinheit (20) von dem Benutzer gesammelt werden, und aus den Daten Informationen zu extrahieren, die für den Zustand des Benutzers derart repräsentativ sind, um die biometrische Erkennung des Benutzers durchzuführen und den Gesundheitszustand des Benutzers auszuwerten.

12. System gemäß einem der vorhergehenden Ansprüche, wobei die Steuereinheit (21) eingerichtet ist, Daten vom EKG-Modul (35) derart zu verarbeiten, um eine biometrische Erkennung des Benutzers durch Vergleichen der vom EKG-Modul (35) empfangenen Daten mit einer Datenbank registrierter Benutzer bereitzustellen.

13. System gemäß einem der vorhergehenden Ansprüche, wobei die Steuereinheit (21) mit einer Anzeigeeinheit (55) verbunden ist, die eingerichtet ist, eine grafische Darstellung und Repräsentation der von der Steuereinheit (21) verarbeiteten Signale, des Ergebnisses des Erkennungsprozesses, und/oder des Ergebnisses der Analyse der Signale bereitzustellen.

14. System gemäß einem der vorhergehenden Ansprüche, wobei die Stromversorgungseinheit (22) eines oder mehrere der folgenden Elemente umfasst: eine Verbindung zum Stromnetz; eine Batterie oder eine andere Energiespeichereinrichtung; einen drahtlosen Empfänger, der in der Lage ist, Energie von einer speziellen drahtlosen Emissionsquelle zu empfangen; einen hydraulischen Generator, der in der Toilette oder in einer anderen Struktur, in der Wasser fließt, installiert ist; einen Empfänger, der in der Lage ist, Energie von WiFi- oder anderen Funkfrequenzsignalen zu empfangen, die von einem externen Sender ausgesendet werden; ein Solarpanel.

15. System gemäß einem der vorhergehenden Ansprüche, wobei jeder Anschluss (52) des Kontaktmessmoduls (31) eine leitende Oberseite (57) für den Kontakt mit dem Körper des Benutzers aufweist und derart positioniert ist, um im Wesentlichen bündig mit einer Oberseite (11) des Toilettensitzes (3) zu sein; wobei das Paar von Anschlüssen (52) derart positioniert ist, dass es, wenn der Benutzer auf dem Toilettensitz (3) in einer normalen Benutzungsposition sitzt, die linken bzw. rechten Oberschenkel des Benutzers berührt; und wobei die Anschlüsse (52) oder zumindest ein Abschnitt davon, der die Oberseite (57) umfasst, aus nichtmetallischen Materialien hergestellt sind, die aus leitfähigen Beschichtungen, leitfähigen Keramiken, leitfähigen Polymermaterialien und Indium-ZinnOxid (ITO) ausgewählt sind.

16. System gemäß Anspruch 15, wobei die Anschlüsse (52) mit Vorsprüngen (60) vorgesehen sind, die von der Oberseite (57) abstehen, um den Kontakt zwischen den Anschlüssen (52) und damit dem Kontaktmessmodul (31) und der Haut des Benutzers zu vergrößern.

17. Verfahren zum Detektieren biomedizinischer Parameter eines auf einem Toilettensitz sitzenden Subjekts, das die folgenden Schritte umfasst:
- Bereitstellen einer Toilettensitzanordnung (2), die zumindest einen Toilettensitz (3) mit einer Sensoreinheit (20) umfasst, die ein oder mehrere elektronische Sensormodule (30) umfasst; wobei die Sensormodule (30) zumindest ein Kontaktmessmodul (31) umfassen, das zumindest einen Sensor (51) aufweist, der eine direkte Schnittstelle mit dem Körper des Benutzers definiert, um EKG-Signale von dem Benutzer zu erfassen, und ein Paar von Anschlüssen (52) umfasst, die auf dem Toilettensitz (3) derart positioniert sind, um in direktem Kontakt mit den jeweiligen Oberschenkeln des Benutzers zu stehen, der in einer normalen Benutzungsposition auf dem Toilettensitz (3) sitzt; wobei das Kontaktmessmodul (31) nur zwei Anschlüsse (52) umfasst, die auf gegenüberliegenden Seiten des Toilettensitzes (3) in Bezug auf eine Mittelachse (A) desselben in Positionen angeordnet sind, die den linken und rechten Oberschenkeln des auf dem Toilettensitz (3) sitzenden Benutzers entsprechen; wobei die beiden Anschlüsse (52) durch ein oder mehrere Isolierelemente (61) getrennt sind, die zwischen den Anschlüssen (52) im Toilettensitz (3) angeordnet sind;
- Detektieren einer elektrischen Potentialdifferenz, die zwischen den beiden Anschlüssen (52) des Kontaktmessmoduls (31) gemessen wird; und Erzeugen eines virtuellen Massesignals, das eine virtuelle Referenzspannung repräsentiert, wodurch die Verwendung von nur zwei Messanschlüssen für die Erfassung von EKG-Signalen ermöglicht wird;
- Verarbeiten der vom Kontaktmessmodul (31) detektierten Signale, um ein Ergebnis bereitzustellen, das eine biometrische Identifizierung des Benutzers und/oder eine Repräsentation der Körperparameter des Benutzers umfasst;
- Anzeigen des Ergebnisses.

18. Verfahren gemäß Anspruch 17, wobei die biometrische Identifizierung des Benutzers kontinuierlich durchgeführt wird, indem kontinuierlich Signale von dem Benutzer erfasst werden und diese Signale über einen Zeitraum verarbeitet werden, in dem der Benutzer auf dem Toilettensitz (3) sitzt.

19. Verfahren gemäß Anspruch 17 oder 18, umfassend einen Schritt des Anpassens der Information, die als Ergebnis des Verfahrens bereitgestellt wird, indem die Durchführung des Verfahrens automatisch an die von dem Verfahren erkannte Identität des Benutzers angepasst wird.

20. Verfahren gemäß einem der Ansprüche 17 bis 19, wobei die biometrische Identifizierung des Benutzers durch die folgenden Schritte erhalten wird: Verarbeiten von Daten von der Sensoreinheit (20) und Vergleichen dieser Daten mit Daten, die in einer Datenbank bekannter Benutzer gespeichert sind, um einen aktuellen Benutzer zu identifizieren.

21. Verfahren gemäß einem der Ansprüche 17 bis 20, wobei die biometrische Identifizierung des Benutzers nacheinander einen Vorverarbeitungsschritt, in dem das von der Sensoreinheit (20) empfangene Signal vorverarbeitet wird, um ein vorverarbeitetes Signal bereitzustellen; einen Extraktionsschritt, in dem für den Benutzer repräsentative Daten aus dem vorverarbeiteten Signal extrahiert werden; und einen abschließenden Klassifizierungsschritt, in dem eine den Benutzer identifizierende Entscheidung erzeugt wird, umfasst.

22. Verfahren gemäß einem der Ansprüche 17 bis 21, wobei der Schritt des Anzeigens des Ergebnisses das Anzeigen eines oder mehrerer der folgenden Elemente umfasst: Identifizierung des Benutzers; Verifizierung der Identität des Benutzers; Darstellung der Entscheidung und der Ergebnisse der biomedizinischen Überwachungsanalyse; Anpassung der Einstellungen an den Benutzer; Benachrichtigungen bezüglich des Gesundheitszustands des Benutzers.

23. Verfahren gemäß einem der Ansprüche 17 bis 22, das einen Schritt des Erfassens und Verarbeitens von einem oder mehreren der folgenden Elemente umfasst: Wärmebilder und/oder Videobilder von zumindest einem Teil des Körpers des Benutzers; mechanische Verschiebungen, die mit der kardiovaskulären Aktivität des Körpers assoziiert werden, indem Funkwellen im GHz-Bereich detektiert werden; PPG (Photoplethysmographie)-Daten;
PCG (Phonokardiographie)-Daten;
fNIRS (funktionelle Nahinfrarotspektroskopie)-Daten;
IMU (Inertial Measurement Unit)-Daten; Lichtwellenleiterdaten.

## Revendications

1. Système pour détecter des paramètres biomédicaux provenant d'un sujet assis sur un siège de toilette, comprenant : un assemblage de siège de toilette (2) comprenant au moins un siège de toilette (3) ; une unité sensorielle (20) comprenant un ou plusieurs modules de capteurs électroniques (30) configurés pour détecter des données provenant d'un utilisateur assis sur le siège de toilette (3) dans une position normale d'utilisation ; une unité de commande (21) connectée à l'unité sensorielle (20) pour recevoir des données à partir de celle-ci ; et une unité d'alimentation (22) pour fournir de l'énergie à l'unité sensorielle (20) et à l'unité de commande (21) ;
dans lequel les modules de capteurs (30) comprennent au moins un module de mesure par contact (31) ayant au moins un capteur (51) définissant une interface directe avec le corps de l'utilisateur pour acquérir des signaux d'ECG provenant de l'utilisateur ; et un module d'ECG (électrocardiographie) (35), configuré pour traiter les signaux d'ECG reçus provenant du module de mesure par contact (31) ;
et dans lequel le capteur (51) du module de mesure par contact (31) comprend une paire de bornes de capteurs (52) positionnées sur le siège de toilette (3) de façon à être en contact direct avec les cuisses respectives de l'utilisateur assis sur le siège de toilette (3) dans une position normale d'utilisation ; le capteur (51) étant configuré pour détecter une différence de potentiel électrique mesurée entre lesdites deux bornes (52) en contact avec le corps de l'utilisateur ;
**caractérisé en ce que** le module de mesure par contact (31) comprend seulement deux bornes (52), positionnées sur des côtés opposés du siège de toilette (3) et d'un axe central (A) de celui-ci, dans des positions correspondant aux cuisses gauche et droite de l'utilisateur assis sur le siège de toilette (3) ; les deux bornes (52) étant séparées par un ou plusieurs éléments isolants (61) positionnés entre les bornes (52) dans le siège de toilette (3) ;
et **en ce que** le module d'ECG (35) est configuré pour effectuer une transduction et un conditionnement de signal avec masse virtuelle des signaux d'ECG reçus provenant du module de mesure de contact (31), le module d'ECG (35) ayant un circuit électronique configuré de façon à générer un signal de masse virtuelle, c'est-à-dire à fournir une tension de référence virtuelle, en permettant l'utilisation de seulement deux bornes de mesure pour l'acquisition de signaux d'ECG.

2. Système selon la revendication 1, dans lequel le module d'ECG (35) a un circuit électronique configuré pour effectuer une détection et une conversion de paramètres physiques, acquis par le module de mesure par contact (31), en signaux, et pour filtrer et amplifier lesdits signaux de façon que lesdits signaux puissent être traités comme une quantité électrique devant être transmise à l'unité de commande (21).

3. Système selon l'une quelconque des revendications précédentes, dans lequel le module d'ECG (35) est disposé sur le siège de toilette (3) à proximité des bornes (52) du module de mesure par contact (31) définissant l'interface avec l'utilisateur.

4. Système selon l'une quelconque des revendications précédentes, dans lequel les deux bornes (52) sont séparées par un ou plusieurs éléments isolants (61) positionnés entre les bornes (52) dans le siège de toilette (3) le long de l'axe central (A).

5. Système selon l'une quelconque des revendications précédentes, dans lequel le circuit électronique du module d'ECG (35) comprend un filtre passe-bande ayant une bande passante comprise entre 0,5 et 40 Hz ; et un amplificateur, réalisant une amplification avec un gain compris entre 10 et 40 000.

6. Système selon l'une quelconque des revendications précédentes, dans lequel l'unité sensorielle (20) comprend un ou plusieurs modules de capteurs électroniques (30) choisis parmi un module de PPG (photopléthysmographie) (36), un module de PCG (phonocardiographie) (37), un module de fNIRS (spectroscopie fonctionnelle dans le proche infrarouge) (38) ; et le module de mesure par contact (31) est configuré pour acquérir, en plus des signaux d'ECG, un ou plusieurs des signaux de PPG, de PCG, et de fNIRS, qui sont ensuite traités par le module d'ECG (35), le module de PPG (36), le module de PCG (37), le module de fNIRS (38) respectivement ; et dans lequel le module d'ECG (35), le module de PPG (36), le module de PCG (37) et le module de fNIRS (38) sont tous connectés au module de mesure par contact (31) pour traiter des données reçues provenant de celui-ci ; chacun parmi le module d'ECG (35), le module de PPG (36), le module de PCG (37) et le module de fNIRS (38) comprenant un circuit électronique configuré pour effectuer une transduction et un conditionnement de signal.

7. Système selon l'une quelconque des revendications précédentes, dans lequel l'unité sensorielle (20) comprend un module d'imagerie thermique (32) et/ou un module d'imagerie vidéo (33) comprenant des caméras respectives ou d'autres éléments d'imagerie (53) configurés pour acquérir des images, des images thermiques ou des images optiques respectivement, provenant de l'utilisateur ; le module d'imagerie thermique (32) et le module d'imagerie vidéo (33) avec les éléments d'imagerie respectifs (53) étant logés dans l'abattant (4) de l'assemblage de siège de toilette (2).

8. Système selon l'une quelconque des revendications précédentes, dans lequel l'unité sensorielle (20) comprend un module d'émetteur-récepteur radio (34) comprenant un ou plusieurs éléments émetteurs-récepteurs radio (54) configurés pour émettre et/ou recevoir des ondes radio dans la gamme des GHz, de façon à détecter des déplacements mécaniques associés à l'activité cardiovasculaire du corps de l'utilisateur.

9. Système selon l'une quelconque des revendications précédentes, comprenant un module de transmission de signaux (46), configuré pour recevoir des données provenant des modules de capteurs (30), soit directement soit converties par un module de conversion analogique-numérique (45), et pour transmettre lesdites données à l'unité de commande (21).

10. Système selon l'une quelconque des revendications précédentes, dans lequel l'unité de commande (21) est une unité de commande dédiée, par exemple une unité de microcontrôleur, soit intégrée dans une quelconque partie de l'assemblage de siège de toilette (2), par exemple dans le siège de toilette (3), soit située à une position éloignée ; ou bien l'unité de commande (21) est intégrée dans une autre unité de commande centrale d'une toilette ou d'une autre installation sanitaire dans laquelle le système (1) est intégré ; ou bien l'unité de commande (1) est intégrée dans un dispositif électronique portable tel qu'une tablette informatique, un téléphone portable, etc.

11. Système selon l'une quelconque des revendications précédentes, dans lequel l'unité de commande (21) est configurée pour traiter des données reçues provenant de l'unité sensorielle (20) et collectées en temps réelles par l'unité sensorielle (20) à partir de l'utilisateur, et pour extraire à partir desdites données des informations représentatives de l'état de l'utilisateur de façon à effectuer la reconnaissance biométrique de l'utilisateur et à évaluer l'état de santé de l'utilisateur.

12. Système selon l'une quelconque des revendications précédentes, dans lequel l'unité de commande (21) est configurée pour traiter des données provenant du module d'ECG (35) de façon à réaliser une reconnaissance biométrique de l'utilisateur en comparant lesdites données reçues provenant du module d'ECG (35) avec une base de données d'utilisateurs enregistrés.

13. Système selon l'une quelconque des revendications précédentes, dans lequel l'unité de commande (21) est associée à une unité d'affichage (55) configurée pour offrir une présentation graphique et une représentation des signaux traités par l'unité de commande (21), du résultat du traitement de reconnaissance, et/ou du résultat de l'analyse des signaux.

14. Système selon l'une quelconque des revendications précédentes, dans lequel l'unité d'alimentation (22) comprend un ou plusieurs parmi : une connexion au réseau ; une batterie ou un autre dispositif de stockage d'énergie ; un récepteur sans fil capable de recevoir de l'énergie provenant d'une source d'émission sans fil dédiée ; un générateur hydraulique installé dans la toilette ou dans une autre structure où circule de l'eau ; un récepteur capable de recevoir de l'énergie provenant du WiFi ou d'autres signaux de radiofréquence émis par un émetteur externe ; un panneau solaire.

15. Système selon l'une quelconque des revendications précédentes, dans lequel chaque borne (52) du module de mesure de contact (31) a une surface supérieure conductrice (57) destinée à venir en contact avec le corps de l'utilisateur et positionnée de façon à être pratiquement en affleurement avec une surface supérieure (11) du siège de toilette (3) ; la paire de bornes (52) étant positionnées de façon à venir au contact, quand l'utilisateur est assis sur le siège de toilette (3) dans une position normale d'utilisation, des cuisses gauche et droite respectivement de l'utilisateur ; et dans lequel les bornes (52) ou au moins une portion de celles-ci comprenant la surface supérieure (57) sont faites de matériaux non métalliques choisis parmi les revêtements conducteurs, les céramiques conductrices, les matériaux polymères conducteurs, l'oxyde d'indium et d'étain (ITO).

16. Système selon la revendication 15, dans lequel les bornes (52) sont dotées de saillies (60), qui dépassent de la surface supérieure (57) pour augmenter le contact entre les bornes (52) et donc le module de mesure par contact (31), et la peau de l'utilisateur.

17. Procédé pour détecter des paramètres biomédicaux provenant d'un sujet assis sur un siège de toilette, comprenant les étapes de :
- fourniture d'un assemblage de siège de toilette (2), comprenant au moins un siège de toilette (3), avec une unité sensorielle (20) comprenant un ou plusieurs modules de capteurs électroniques (30) ; les modules de capteurs (30) comprenant au moins un module de mesure par contact (31) ayant au moins un capteur (51) définissant une interface directe avec le corps de l'utilisateur pour acquérir des signaux d'ECG provenant de l'utilisateur et comprenant une paire de bornes (52) positionnées sur le siège de toilette (3) de façon à être en contact direct avec les cuisses respectives de l'utilisateur assis sur le siège de toilette (3) dans une position normale d'utilisation ; dans laquelle le module de mesure par contact (31) comprend seulement deux bornes (52), positionnées sur des côtés opposés du siège de toilette (3) et d'un axe central (A) de celui-ci, dans des positions correspondant aux cuisses gauche et droite de l'utilisateur assis sur le siège de toilette (3) ; les deux bornes (52) étant séparées par un ou plusieurs éléments isolants (61) positionnés entre les bornes (52) dans le siège de toilette (3) ;
- détection d'une différence de potentiel électrique mesurée entre lesdites deux bornes (52) du module de mesure par contact (31) ; et génération d'un signal de masse virtuelle, représentatif d'une tension de référence virtuelle, permettant l'utilisation de seulement deux bornes de mesure pour l'acquisition de signaux d'ECG ;
- traitement des signaux détectés par le module de mesure par contact (31) pour présenter un résultat comprenant une identification biométrique de l'utilisateur et/ou une représentation de paramètres corporels de l'utilisateur ;
- affichage du résultat.

18. Procédé selon la revendication 17, dans lequel l'identification biométrique de l'utilisateur est effectuée en continu, par acquisition de signaux provenant de l'utilisateur et traitement des signaux, en continu sur la période de temps pendant laquelle l'utilisateur est assis sur le siège de toilette (2).

19. Procédé selon la revendication 17 ou 18, comprenant une étape de personnalisation des informations obtenues en résultat du procédé, par ajustement automatique de la mise en œuvre du procédé en fonction de l'identité de l'utilisateur tel que reconnu par le procédé.

20. Procédé selon l'une des revendications 17 à 19, dans lequel l'identification biométrique de l'utilisateur est obtenue par les étapes de : traitement de données provenant de l'unité sensorielle (20) et comparaison desdites données avec des données stockées dans une base de données d'utilisateurs connus, de façon à identifier l'utilisateur actuel.

21. Procédé selon l'une des revendications 17 à 20, dans lequel l'identification biométrique de l'utilisateur comprend, en séquence, une étape de pré-traitement, dans laquelle le signal reçu provenant de l'unité sensorielle (20) est prétraité pour offrir un signal prétraité ; une étape d'extraction, dans laquelle des données représentatives de l'utilisateur sont extraites du signal prétraité ; et une étape de classification finale, dans laquelle il est produit une décision identifiant l'utilisateur.

22. Procédé selon l'une des revendications 17 à 21, dans lequel l'étape d'affichage du résultat comprend l'affichage d'une ou plusieurs parmi : l'identification de l'utilisateur ; la vérification de l'identité de l'utilisateur ; la présentation de décision et de résultats de l'analyse de surveillance biomédicale ; la personnalisation de réglages pour l'utilisateur ; les notifications concernant l'état de santé de l'utilisateur.

23. Procédé selon l'une des revendications 17 à 22, comprenant une étape d'acquisition et de traitement d'un ou plusieurs parmi :
des images thermiques et/ou des images vidéo d'au moins une partie du corps de l'utilisateur ;
des déplacements mécaniques associés à l'activité cardiovasculaire du corps par détection d'ondes radio dans la gamme des GHz ;
des données de PPG (photopléthysmographie) ;
des données de PCG (phonocardiographie) ;
des données de fNIRS (spectroscopie fonctionnelle dans le proche infrarouge) ;
des données d'IMU (unité de mesure inertielle) ;
des données de fibres optiques.
